(19) Europäisches Patentamt European Patent Office Office européen des brevets

(11) **EP 3 691 549 B1**

(12) **EUROPEAN PATENT SPECIFICATION**

(45) Date of publication and mention
of the grant of the patent:
**16.07.2025 Bulletin 2025/29**

(51) International Patent Classification (IPC):
**A61B 17/135** (2006.01)  **A63B 21/00** (2006.01)
**A63B 23/04** (2006.01)  **A63B 23/12** (2006.01)
**A63B 24/00** (2006.01)  **A61B 5/00** (2006.01)
**A63B 71/06** (2006.01)  **A61B 17/00** (2006.01)

(21) Application number: **18864029.6**

(22) Date of filing: **05.10.2018**

(52) Cooperative Patent Classification (CPC):
**A61B 17/1355; A61B 5/6824; A63B 24/0087;**
**A63B 71/0622;** A61B 2017/00017;
A61B 2017/00106; A61B 2017/00221;
A61B 2017/00734; A63B 24/0075; A63B 2024/0093;
A63B 2071/0625; A63B 2071/065; A63B 2071/0661;
A63B 2209/10; A63B 2220/56; (Cont.)

(86) International application number:
**PCT/AU2018/051078**

(87) International publication number:
**WO 2019/068147 (11.04.2019 Gazette 2019/15)**

(54) **BLOOD OCCLUSION OR RESTRICTION CUFF**

BLUTVERSCHLUSS ODER -BEGRENZUNGSMANSCHETTE

MANCHON D'OCCLUSION OU DE RESTRICTION DE SANG

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **06.10.2017 AU 2017904033**

(43) Date of publication of application:
**12.08.2020 Bulletin 2020/33**

(73) Proprietor: **Saga Fitness Pty Ltd**
**Newstead, QLD 4006 (AU)**

(72) Inventor: **MARCUS, Joseph**
**Mermaid Beach**
**New South Wales 4218 (AU)**

(74) Representative: **Meissner Bolte Nürnberg**
**Patentanwälte Rechtsanwälte**
**Partnerschaft mbB**
**Bankgasse 3**
**90402 Nürnberg (DE)**

(56) References cited:
**WO-A1-2016/087123  WO-A1-2016/148956**
**WO-A1-2017/149690  CN-U- 204 909 555**
**US-A1- 2009 318 818  US-A1- 2012 065 561**
**US-A1- 2013 211 269  US-A1- 2014 159 912**
**US-A1- 2016 120 445  US-A1- 2016 193 491**
**US-B2- 8 366 740**

(52) Cooperative Patent Classification (CPC): (Cont.)
A63B 2220/80; A63B 2220/802; A63B 2225/50;
A63B 2225/62; A63B 2230/06; A63B 2230/207

**Description**

**TECHNICAL FIELD**

The present invention relates to the activity of body building, weight training as well as blood flow control in the medical field. In a preferred form, the invention comprises a smartphone application (app) operated fitness apparatus used to restrict venous blood flow to promote muscular strength and hypertrophy.

Furthermore, the invention can be used as a self-contained and wireless medical tourniquet to prevent critical blood loss and hypoxic tissue damage in emergency and traumatic life threatening situations.

The improvement of the invention is directed to removing the necessity of various inflation tubes and electrical cables associated with prior art cuffs and tourniquets, which not only restrict movement during training, but also require ancillary equipment such as external air pumps and monitoring devices required for their operation.

**BACKGROUND TO THE INVENTION**

While the invention is directed to an improved self-contained cuff and tourniquet which dispenses with the requirement of lines, tubing or equipment currently associated with such devices, it is instructive to examine their present use and the state of the prior art.

*Blood Flow Restriction Training*

According to conventional theory, to induce muscular hypertrophy, lifting weights with a minimum of 70% of one repetition maximum tests (1RM) is needed. It is accepted that intensities below this threshold lack the essential stimulus to produce muscular adaptations in the sarcoplasms and myofibrillars of muscle tissue *(Peterson et al 2011)*. Recent studies, however, using relatively low loads of predicted 1RM as low as 20% combined with blood flow restriction (BFR) have been effective in eliciting muscular hypertrophy and strength similar to that achieved with higher loads *(Abe et al 2005)*.

Prior art blood flow restriction training is a muscular hypertrophy and strength stimulus utilising the application of specially adapted pressure cuffs to the top of a limb e.g. an upper thigh and/or arm which are inflated to a predetermined pressure throughout the duration of the exercise *(Abe et al 2005, Loenneke 2010)*. The pressure applied to the limb is strong enough to partially restrict the venous return of blood from peripheral muscle beds to the heart, but not strong enough to occlude arterial blood supply to the muscle region *(Abe et al 2005, Loenneke 2010)*.

Blood flow restriction (BFR) training should not be confused with training under ischemic conditions. BFR does not induce ischemia within skeletal muscle, but rather promotes a state of blood pooling in the capillaries within the limb musculature as venous return is only slightly impaired and muscle bed perfusion under mean arterial pressure is maintained throughout the training load or exercise regimen *(Abe et al 2005)*.

*Medical Tourniquets*

The use of medical tourniquets is widely documented. In Outpatient clinics, where it is less critical, tourniquets usually comprise elastic or other manually operated bands. They do not allow for accurate pressure levels to be established and are used merely to occlude venous return or in the extreme, arterial blood supply to a limb to control blood loss. These simple occlusion cuffs, often connected to an analogue gauge, usually portable, comprise a hand operated pump which inflates an air cuff. While this is a basic, portable and low cost device, it cannot be used to accurately monitor the perfusion of a muscle bed. Simpler devices, such as blood flow restriction (BFR) bands, usually comprise elastic material to restrict blood flow. They are cheap and versatile but cannot gauge or retain pressure and invariably are not associated with monitoring systems.

In the intensive treatment of patients, where control of the circulatory system is vital, machine operated tourniquets connected to digital pressure monitors and pumps, allow for pressure to be accurately applied by controlled inflation of cuffs which impede blood flow. This is especially important where reperfusion of a damaged limb needs to occur periodically. Such tourniquets have to be programmed to deflate regularly to allow arterial inflow into the affected area to help mitigated the risk of tissue damage from hypoxia. Such prior art reperfusion tourniquets are generally associated with separate pump and control modules found more commonly in the accident and emergency (A & E) or intensive care units (ICU) of hospitals.

Relevant prior art medical tourniquets include the Kaatsu tourniquet, which is used widely in Japan, and comprises a machine with an electronic monitor system connected to air cuffs via plastic tubing. While it has the advantage of an electronic monitor which is relatively portable and accurate, it has external tubing, and therefore not ideal for dynamic exercise or mobile use. The Delphi Blood flow restriction cuff is a digital machine, connected to an intravenous (IV) pole with plastic tubing connected to an air cuff. It can monitor pressure applied to a limb accurately but is expensive, cumbersome, not portable, and needs to be connected to a mains power source. It also requires the use of external tubing which compromises the user's mobility and/or freedom of movement.

WO 2017/149690 A1 discloses a belt having a gas bag and configured to impart to the specific portion a pressurizing force based on the amount of gas in the gas bag in a state of being wrapped around a specific part of the user's limb; configured to control a pressurizing force applied by the belt to the specific portion by controlling a supply amount and a discharge amount of the gas to the gas bag of the belt based on the control command and a portable information terminal device configured to wirelessly communicate the control command to the control device when operated in a predetermined manner.

US 2016/193491 A1 discloses a muscle training method, comprising the following steps that are repeated alternately to perform training of a muscle of a user: a pressuring and exercise step of winding a belt around at least one of four limbs of the user and applying specific pressure thereto so as to restrict blood circulation of the muscle of the user without stopping the blood circulation, and asking the user to perform load-applied exercise to apply load of specific weight to the muscle of the user; and an exercise stopping step of asking the user to stop the load-applied exercise while continuously applying the specific pressure to the user, wherein the specific weight is set at a value smaller than maximum weight necessary for the user to exert maximum muscle force.

US 8,366,740 B2 discloses system and a method for controlling blood flow through a zone of a patient limb, the zone being bounded by a proximal end and a distal end. The system comprises a cuff configured for securing to the limb and for covering the zone, the cuff being inflatable to provide pressure to the zone for occluding the flow of blood flowing through the zone in the direction from the proximal to distal ends of the zone, an array of sensors fitting between the cuff and the limb and arranged for sensing and signalling a distance from the proximal end that the arterial blood flow penetrates into the zone; and a control instrument connected to the array and to the cuff for regulating the pressure in the cuff to occlude the blood flow in the zone depending upon the arterial blood flow penetration distance signalled by the array; and an inflatable auxiliary bladder disposed adjacent to the array and inflatable independently from the cuff for varying an angular position of the sensors of the array relative to the limb while the zone is covered with the inflatable cuff secured to the limb to provide pressure to the zone, thereby to scan various tissue volumes.

US 2016/120445 A1 discloses apparatuses and methods for monitoring the cardiac health of a patient, which incorporates secondary information. The apparatus may be a heart rate monitor, a blood pressure monitor, an ECG or other biometric device in communication with an electronic device having communication ability. The devices detect a cardiac or other biometric measurement and correlate the detected information with secondary information, such as GPS data, to produce a contextualized measurement.

US 2013/211269 A1 discloses a limb occlusion device capable of performing more than one function, for example as a limb occlusion tourniquet, as a blood pressure monitor and as a device for automatic delivery of remote conditioning treatment.

WO 2016/148956 A1 discloses methods and devices for delivery of a remote ischemic conditioning treatment. The device comprises a cuff configured for placement over the limb of the subject and a controller operably connected to said cuff, wherein said cuff is further configured to at least partially reduce blood flow in said limb upon inflation thereof.

It is therefore an advantage to provide a versatile venous restriction or occlusion cuff without any attached tubing or cables for use in muscular strength and hypertrophy training, but which can also be used as an arterial reperfusion tourniquet to stop traumatic blood loss without tissue hypoxia at increased cuff pressures.

It is also an advantage that the invention is a standalone and relatively compact apparatus which allows for greater user mobility while training and which reduces the amount of space taken up in storage or transport.

Importantly, the invention seeks to provide the public with a commercial alternative and useful improvement over the prior art.

SUMMARY OF THE INVENTION

The invention is set forth in the independent claims. Embodiments result from the dependent claims and the below description.

In one aspect therefore, the invention resides in a remote controlled blood flow restriction cuff as defined in independent claim 1.

Preferably, the controller enables the cuff to be inflated to and retained at a pressure up to 350 mmHg. It will be obvious that an inflation pressure above that of a systolic pressure of a wearer will facilitate the cuff to be used as medical tourniquet to stop blood flow.

Preferably, the air pump is a battery operated air pump.

Preferably, the power supply is a rechargeable battery power supply.

Preferably, the air pump, power supply, pressure sensor, and controller are housed in a module proximal to the air bladder, which are all located on the cuff.

Preferably, the operation of the cuff is controlled remotely over a wireless protocol such as Bluetooth, associated with a Smart computer or phone application or other equivalent system.

Preferably, the pressure sensor detects pressure to the nearest 1 mmHg, wherein pressure in the cuff is maintained at the predetermined pressure level by the wireless controller operating the air pump responsive to signals from the pressure sensor.

Suitably, the controller accounts for variance in pressure due to limb movement for the duration required until the pressure is released from the cuff.

In another aspect, the invention resides in a method of using a remote controlled blood flow restriction cuff as defined in independent claim 15.

Suitably, where available or desired, a pulse oximeter can be used in the alternative or in conjunction with a Doppler ultrasound to monitor arterial inflow.

It will be obvious, the advantage of using oximetry would be a readily obtainable pulse rate and a blood oxygen saturation level.

The present disclosure provides a digital tourniquet device with no external wires or tubing, and is a fully self-enclosed pneumatic air cuff. They are designed to be used as medical tourniquets during trauma events to stop blood flow and to be used in fitness during blood flow restriction training.

They are blood flow restriction devices which are digital, wireless, and pneumatic cuffs, to be worn on the upper limbs and lower limbs. The blood flow restriction or occlusion device of the present invention is electronic, completely wireless and tubeless design that includes an integrated power supply. The device is self-contained, but interacts with an untethered controller that is adapted to be worn on the limbs of a user.

The device measures individualised pressure zones of a user, which are based on personalised physiological data, and maintains a restriction pressure that is exerted upon the limb, which is applied consistently in real time, and accounts for movement of limb. This means that the device makes adjustments to increase/decrease pressure in order to maintain the preselected pressure in accordance with the individualised pressure zone of the user.

The physiological data can include blood flow, pulse rate, blood pressure, limb occlusion pressure (LOP) and arterial occlusion pressure (AOP). The physiological data for blood flow and pulse rate are collected via the following methods, which can be determined by integrated and off the shelf devices coupled with smartphone apps;

- Photo plethysmography
- Bio impedance
- Near Infrared Spectroscopy (NIRS)
- Doppler Ultrasound

Examples

_Arterial Occlusion Pressure_

Arterial occlusion pressure (AOP) is a measure of the cuff pressure required to maintain a bloodless surgical field.

The device of the present disclosure performs the following calculations

1. Measures Systolic Blood Pressure (SBP)
2. The user then inputs their limb circumference data into the controller
3. The circumference data of the limb is used to calculate the corresponding estimated KTP coefficient as based on the following table:-

Table 2.    Tissue padding coefficients based on limb circumferences [28]

| Extremity circumferences (cm) | Estimated $K_{tp}$ |
|---|---|
| 20 | 0.91 |
| 21 | 0.90 |
| 22 | 0.89 |
| 23 | 0.88 |
| 24 | 0.87 |
| 25 | 0.86 |
| 26 to 27 | 0.85 |
| 28 | 0.84 |
| 29 | 0.83 |
| 30 to 31 | 0.82 |
| 32 to 33 | 0.81 |
| 34 | 0.80 |
| 35 to 36 | 0.79 |
| 37 to 38 | 0.78 |
| 39 to 40 | 0.77 |
| 41 to 43 | 0.76 |
| 44 to 45 | 0.75 |
| 46 to 48 | 0.74 |
| 49 to 51 | 0.73 |
| 52 to 54 | 0.72 |
| 55 to 57 | 0.71 |
| 58 to 60 | 0.70 |
| 61 to 64 | 0.69 |
| 65 to 68 | 0.68 |
| 69 to 73 | 0.67 |
| 74 to 75 | 0.66 |

$K_{tp}$: Tissue padding coefficient.

4. The arterial occlusion pressure is calculated according to the following:-

$$\frac{\text{Systolic Blood Pressure} + 10}{\text{KTP coefficient}}$$

5. Then the user is then provided four options to select;

- 50% of total pressure

- 60% of total pressure

- 70% of total pressure

- 80% of total pressure

6. The cuff is then inflated and maintained at the selected % of AOP, and the device keeps this pressure constant, allowing for adjustments with limb/body movement.

*Limb Occlusion Pressure*

Limb occlusion pressure or LOP can be defined as the minimum pressure required to stop the flow of arterial blood into the limb distal to the cuff. LOP is determined by gradually increasing tourniquet pressure until distal blood flow is interrupted.

The risk of tourniquet related complications can be significantly reduced by measuring the LOP and selecting cuff inflation pressures accordingly. Best practice recommends that optimal cuff pressure should be based on the patient's LOP. The tourniquet pressure should be minimized, lower pressures are thought to prevent injury of normal tissue.

The device of the present invention calculates the limb occlusion pressure (LOP) in accordance with the following steps :-

1. The user opens the controller

2. The user places the device on a limb

3. The device uses the following methods to monitor blood flow in the user's extremity;

- Photoplethysmography
- Bioimpedance
- Near *Infrared* Spectroscopy (NIRS)
- Doppler ultrasound

4. The device inflates in increments of 5-10mmhg until pulse stops

5. The device senses at what pressure level pulse rate is no longer detected using aforementioned methods of measuring blood flow and pulse

6. The point at which minimal pressure required to stop blood flow to extremity to limb is called Limb Occlusion Pressure (LOP)

7. The blood flow restriction parameters are based upon LOP

8. The cuff will inflate to desired percentage of LOP 50-80% based on user selection

9. The user begins use of device with selected pressure of LOP

10. The cuff is then inflated and is maintain at selected % of LOP, device keeps pressure constant in this zone, allowing for adjustments with limb/body movement.

*LOP Measurement*

Individual limb occlusion pressure will be measured manually by (a) palpation, and (b) Doppler ultrasound (c) using a distal photo plethysmography sensor (d) NIRS(e) bio impedance .

Tourniquet cuff is automatically inflated and utilizes aforementioned probes to detect arterial pulsations in limb at level of cuff and distal to the tourniquet cuff.

*Benefits of Calculating LOP*

When measured correctly in applying LOP in BFR using surgical grade automated devices, LOP helps in delivering optimum results in rehabilitation by personalizing blood flor restriction training for rehabilitation of elderly, improving performance of injured athletes and in patients recovering from major surgical procedures such as knee arthroscopy.

The devices of the present invention are inflated to a pressure of between 0-350mmhg. There is an on-board module housing the battery operated air pump. The on-board pressure sensor or monitor detects the desired pressure to the nearest 1mmHg. Pressure is maintained at a desired pressure level for the duration needed and is corrected for variance in pressure with limb movement. Pressure is then released by the user. The device is preferably controlled via Bluetooth or wireless controller with accompanying Smartphone application. The devices are to be used in conjunction with and without low load resistance training to increase muscular size and strength comparatively to exercising with higher loads.

Exercising at necessary intensity can cause injury, and people who would benefit most from exercise often cannot participate e.g. injured or sick patients. Typically, to achieve positive muscular adaptations, individuals have to exercise at above 80% of their maximum level. According to literature, however, when low load exercise of 10-30% of maximum is combined with blood flow restriction, the same results to non-blood restriction training can be achieved in comparative time frames.

Currently on the market, there exists two extreme versions of the prior art. These include cumbersome, wired, heavy, medical devices, which are not portable and easily used, or expensive elastic tourniquets, which are not accurate and offer no physiological data, and which are prone to abuse by over tightening.

**BRIEF DESCRIPTION OF THE DRAWINGS**

Preferred embodiments of the invention will now be described, by way of example only, with reference to the accompanying drawings in which:

Figures 1 to 4 show various views of a preferred blood flow restriction cuff in accordance with the invention.

Figure 5 shows the embodiment of Figures 1 to 4 in use as an exercise cuff.

Figure 6 shows the embodiment of Figures 1 to 4 in use as a medical tourniquet.

## DETAILED DESCRIPTION OF PREFERRED EMBODIMENTS

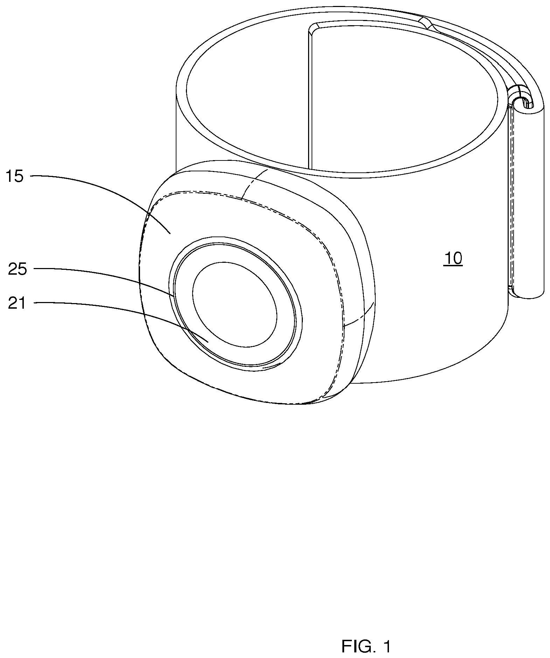

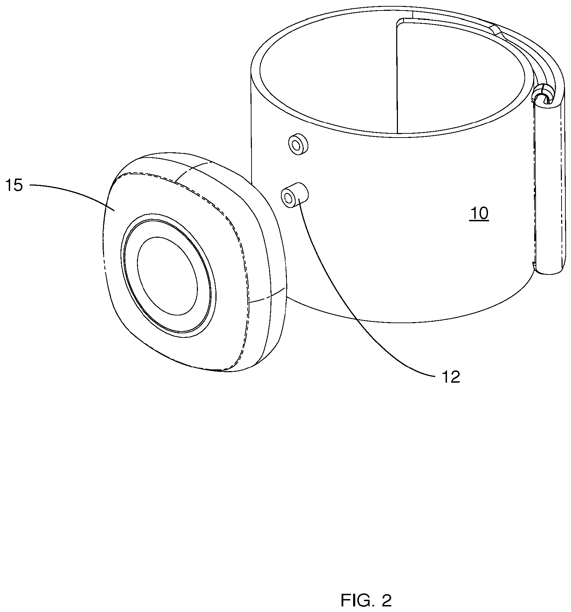

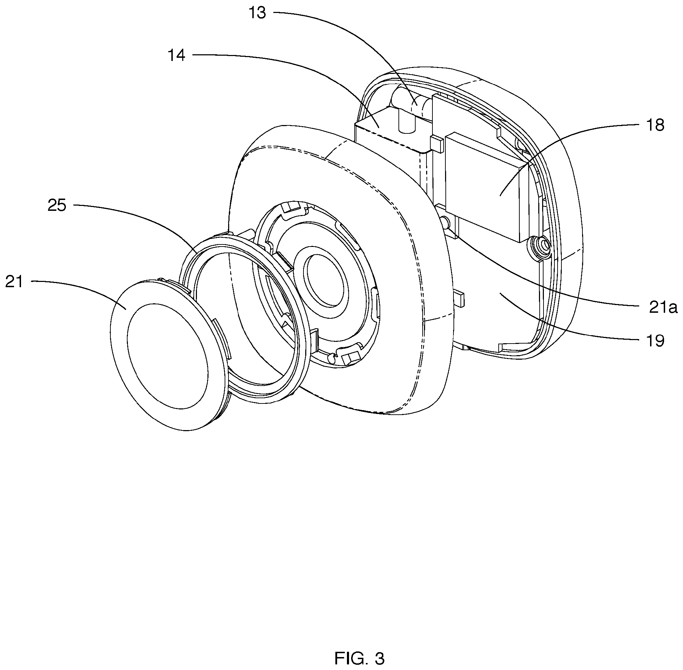

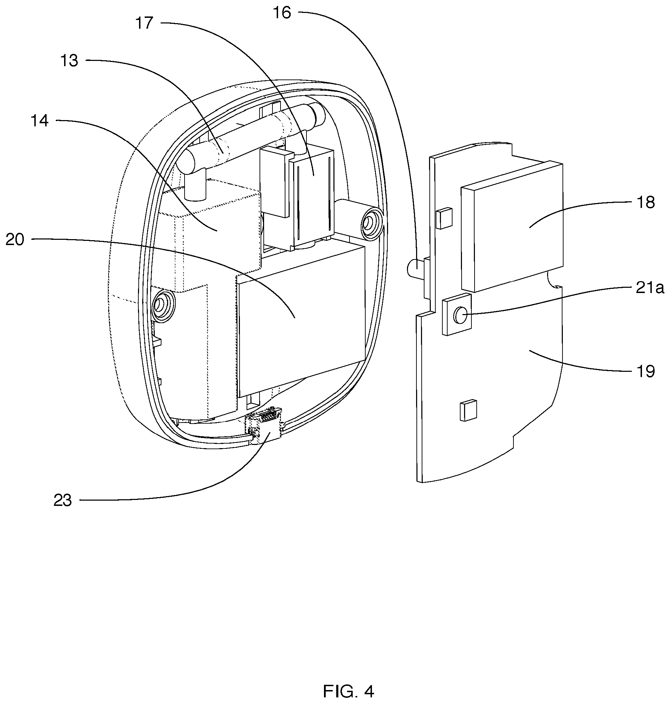

Figures 1 to 4 show views of a preferred remote controlled blood flow restriction cuff 10 according to the invention. Cuff 10 is configured to be positioned around a limb (not shown). Compact air pump 14 in module 15 pressurises the air bladder (not visible as sewn inside the cuff) via tube 13 connected to valve 17 which supplies air to the bladder through air inlet 12. Pressure sensor 16 on PCB board 19 senses the air pressure in the air bladder (not visible as sewn inside the cuff). Microprocessor based electronic controller 18 on PCB board 19 with reset button 21 and reset switch 21a controls operation of the air pump 14 and is adjustable for a predetermined pressure or pressure range. Microprocessor controller 18 is receptive and responsive to signals received from pressure sensor 16.

Power supply 20 is located on the cuff to power the air pump 14 and the controller 18, wherein inflation and deflation of the air bladder is controlled using a remote device such as a Smartphone (not shown) in communication with the controller 18.

As previously mentioned preferably, controller 18 enables the cuff to be inflated to and retained at a pressure up to 350 mmHg. Which will be an inflation pressure above that of systolic pressure of most users which will also facilitate the cuff to be used as medical tourniquet to stop arterial blood loss. Air pump 14 is battery operated and power supply 20 is a rechargeable battery power supply.

As is shown, air pump 14, power supply 20, pressure sensor 16, and controller 18 are housed in a module 22 on cuff 10.

In the preferred embodiment, operation of the cuff 10 is controlled remotely over a wireless protocol such as Bluetooth, associated with a Smart computer or phone application or other equivalent wireless system for example a USB programmed memory stick (not shown) inserted in USB port 23.

Preferably, pressure sensor 16 detects pressure to the nearest 1mmHg, wherein pressure in the air bladder is maintained at the predetermined pressure level by controller 18 operating the air pump 14 responsive to signals from pressure sensor 16.

Suitably, controller 18 accounts for variance in pressure due to limb movement for the duration required until the pressure is released from the air bladder. Circular LED light 25 shows that the power supply 20 has been turned on.

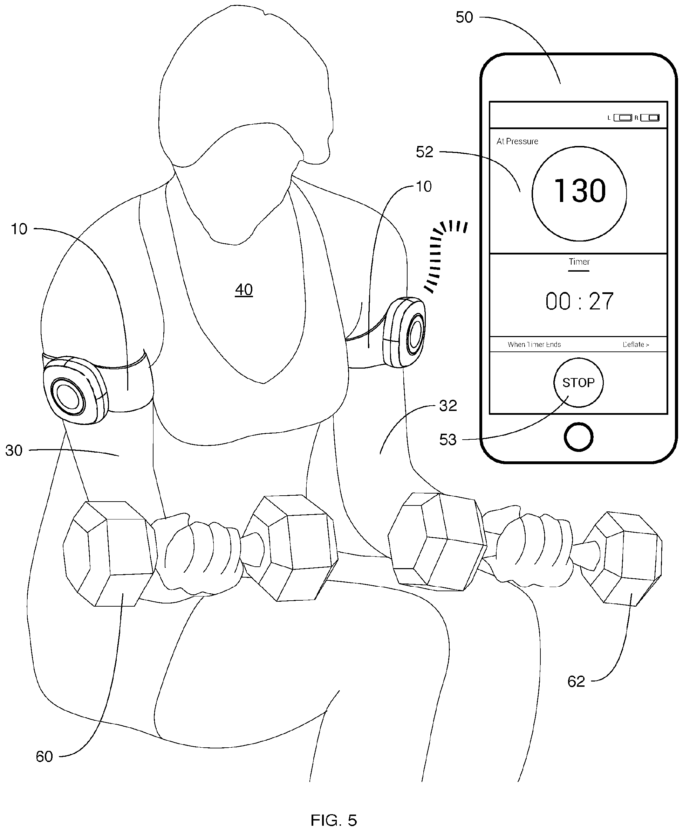

Figure 5 shows the embodiment of Figures 1 to 4 in use as training cuffs 10 to build and strengthen muscle, in this case, the upper arms 30, 32 by lifting dumbbells 60, 62.

The cuffs 10 are applied to the upper arms 30, 32. User 40 initialises and connects with the controller of the cuff via a smartphone 50 with an application (app) 52 running on a Bluetooth protocol.

When connected, the user 40 in response to a pre exercise questionnaire, inputs through the app 52, physiological data, age, height, medical history, gender, limb circumference size, body fat level, heart rate and blood pressure and any other requisite pre exercise information required.

The user 40 then selects the most appropriate training program and a range of cuff pressures and sets a timer for the selected training program. The user presses a start button 53 displayed by the app.

The cuffs 10 inflate to the desired pressure and through the electronic controller in response to a pressure sensor controls the air pump which maintains pressure by inflating and deflating the cuff as necessary to account for any limb movement.

Once the timer has expired or the user stops the program, the cuff deflates, and Smartphone app 52 records cuff pressures and duration in a user profile.

As the smart phone application monitors physiological data continuously and wirelessly via Bluetooth (arterial inflow, pulse rate and oxygen saturation) this physiological data is put through an algorithm in conjunction with age, weight, limb circumference, and lean fat-free mass. This information is used to create a personalised user profile to establish the safest pressure parameters as a percentage of 'Limb Occlusion Pressure' and is customised to each user's needs as a hypertrophy/strength inducing blood flow restriction training device or as a life preserving medical tourniquet device (see below). In the training environment, the smart phone application will store data per session, pressure used, duration, and physiological data which can be used to create a customised user blood flow restriction exercise program.

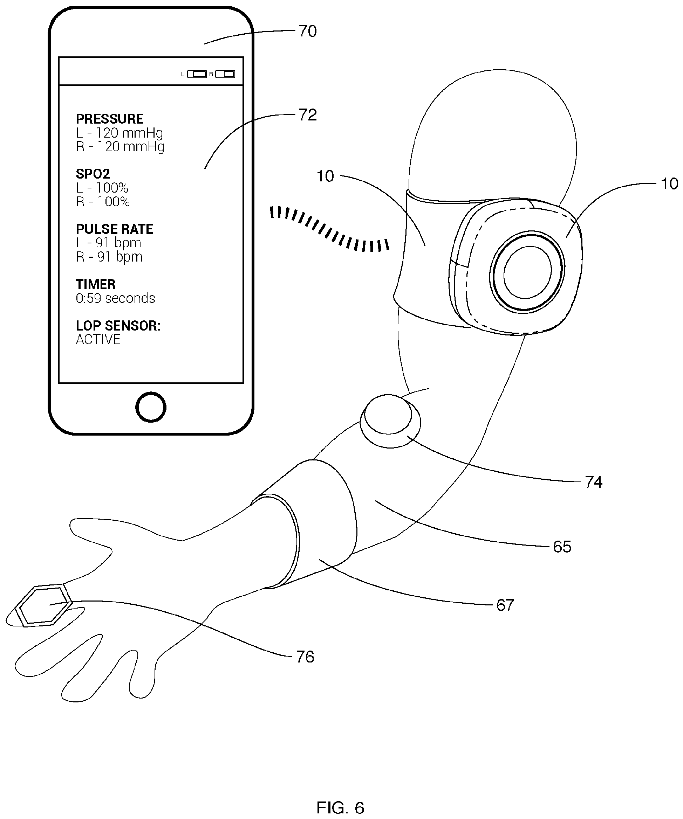

Figure 6 shows the embodiment of Figures 1 to 4 in use as a medical tourniquet.

Cuff 10 is applied to an injured limb 65 with a wound dressed by bandage 67. A user initialises and connects with the controller of the cuff via a smartphone 70 with an application (app) 72 running on a Bluetooth protocol. Arterial inflow to the limb 65 is monitored via a Doppler ultrasound pickup 74. The user then increases cuff pressure, via the app, to an arterial occluding pressure indicated by the attenuation of the Doppler signal. The controller automatically maintains the arterial occluding cuff pressure measured by the pressure sensor by inflating or deflating the cuff in response to the Doppler signal.

The user rates the level of traumatic injury to a limb via the app, wherein a reperfusion program is selected to instruct the controller to enable brief periods of cuff deflation and re-inflation for reperfusion of the limb thereby mitigating the risk of unnecessary hypoxic tissue damage.

The controller monitors arterial inflow wherein the cuff is only deflated and re-inflated in response to the Doppler signal to minimise blood loss in accordance with the reperfusion program. A digital pulse oximeter 76 can also be used to monitor blood oxygen saturation and heartrate.

Once full medical or hospital intervention can be provided, the cuff can then be deflated and removed under medical supervision.

The medical tourniquet cuff and application utilises a smart phone or wireless interface display unit driven system of blood flow restriction achieved by using completely self-contained digital air pneumatic cuffs to impede venous arterial return through a self-regulating pressure sensor including blood flow restriction or occlusion monitoring via continuous wave Doppler ultrasound and/or pulse oximetry.

Continuous wave Doppler ultrasound has the following main function:

By sending and receiving ultrasound waves through the skin located over an artery or heart, when the waves are reflected from a moving object, such as blood passing through an artery, the reflected frequency changes slightly. This change is then analysed by the electronics of the Doppler ultrasound unit and converted into a digital display of the cardiac output and heart rate.

A pulse oximeter has two main functions, which are as follows:

To provide an audible signal of pulsed blood flow, similar to that of a Doppler ultrasound. When an artery is occluded by a pressure cuff, there is a loss of signal from the oximeter; and to measure the oxygen saturation of haemoglobin in blood or tissue.

Continuous wave Doppler ultrasound and the pulse oximetry are used in the measurement of pulse wave transit time distal to position of the cuff on the limb. Pulse wave transit time is defined as the time required for an arterial pulse wave to propagate along a fixed path, which is used to determine 'Limb Occlusion Pressure'. This measurement can be made by monitoring the pulse at a point distal to the application point of the tourniquet. Once the distal pulse rate is absent at the most minimal arterial pressure, this is determined to be the 'Limb Occlusion Pressure'.

The following include examples of procedures which can be adopted in the use of the invention.

First time use and creation of personalised Smartphone Application User Profile.

Explain the procedure to the patient;

Ensure he/she is lying comfortably in a semi-recumbent position.

*Stage 1 - Measuring Upper Limb pressure using the pulse oximeter.*

Place an appropriate size blood-pressure cuff around the patient's upper arm;

Place the pulse oximeter sensor on any finger (Fig 1a). When the sensor is placed on the digit, the oximeter will display two numbers: the first represents the patient's heart rate, the second the percentage of circulating oxygenated haemoglobin. Pulse blood flow is also displayed on the oximeter, either by a waveform or by a column of lights;

Record a baseline reading. Inflate the cuff to 60mmHg, then inflate it in 10mmHg increments, allowing approximately 10 seconds between these increments. Once the pressure reaches 100mmHg, the incremental changes can be increased to 20mmHg;

Record the pressure reading that is one below the point where the audible or pulse signal is lost on the pulse oximeter; for example, if the signal is lost at 180mmHg, record a pressure of 160mmHg;

Repeat the measurement on the other arm, then calculate the 'Limb Occlusion Pressure' by using the higher of the two readings

*Stage 2 - Measuring Lower Limb pressure using the pulse oximeter.*

Place an appropriate size cuff around the largest most proximal thigh region.

Place the oximeter sensor on one of the first three toes (Fig 1b).

Inflate the cuff as outlined in Stage 1, and record the pressure at which the signal is lost.

Repeat the measurement on the other leg, then calculate the 'Limb Occlusion Pressure' index by using the higher of the two readings.

The pressure at which the arterial pulse is stopped corresponds to the minimum tourniquet cuff pressure to occlude the underlying arteries or 'Limb Occlusion Pressure' at that time.

After the 'Limb Occlusion Pressure' is identified, this data is fed through the accompanying Smartphone application. Working pressures for blood flow restriction are personalised using the 'Limb Occlusion Pressure' data gathered. Each user's physiological details are stored in the Smartphone application. The micro processer (or controller) and the pressure sensor/pump implements the calculated pressure target as a percentage of the 'Limb Occlusion

**EP 3 691 549 B1**

Pressure'. This personalised approach ensures only the most minimal blood impedance pressures are applied, helping to mitigate the risks associated with tourniquet application. This ensures arterial inflow into a limb but restricts venous return, which leads to a cascade of physiological benefits as earlier mentioned.

The present invention allows individuals to engage in blood flow restriction training more safely and accurately. It has all the safety and physiological data of expensive wired medical devices but with the added freedom of having all electronics housed in an on-board module with no external tubing or wiring.

It is worn on the upper and lower limbs. Preferably, the cuffs are 5cm or 10 cm wide pneumatic cuffs comprising of an outer cuff material of leather or silicone with airbag contained between the material layers. The cuffs are preferably applied using a ratchet or buckle or Velcro loop fastening system. The pneumatic cuff for an upper limb is 25cm to 50cm length and for a lower limb is 50 to 75cm length.

The airbag is connected to the module unit with a two way valve which can be closed to allow pressure to be held and maintained at a desired pressure level with the pressure sensor and controller maintaining this pressure irrespective of limb movement by making small adjustments in air pressure to allow maintenance of the desired pressure. The cuff module contains an on-board battery, pressure sensor and air pump all housed in a plastic casing which allows the airbag to be inflated and to increase pressure (mmHg) in the airbag. This increase in pressure in mediated by the on-board sensor which increases pressure to a predetermined level between 0-350 mmHg and maintains the set pressure for the duration of use.

The system then allows pressure to be released when desired by the user. Communication with the device is via Bluetooth or an equivalent wireless protocol. The device will have accompanying smart phone application or a wireless remote control unit. There is no external tubing or wiring. The device is completely free and is only attached to a limb by the releasable fastening system.

In summary, the invention can be described as a wireless operated tubeless air pneumatic cuff system with a dual purpose role in blood flow restriction training and as a portable tourniquet for medical emergencies. As a wireless operated tourniquet, it enables pressure exerted on limb to be maintained at a desired level through an inbuilt pressure sensor and controller. The main advantage of the device is that it is completely wireless, portable and self-contained without requiring extraneous tubing connected to ancillary equipment. All electronics and the air bladder are encased within the cuff itself. The device has application in the medical field by being used as a digital medical tourniquet to stop traumatic blood loss at higher cuff pressures as well as in the fitness industry in conjunction with low load resistance training to increase muscle strength and hypertrophy.

It is conveniently operated via a Smartphone application which reacts to physiological data including arterial pulse rate and blood oxygen saturation levels to create a user profile which can then be programmed to automatically control occlusion pressure levels according to customised training protocols.

In this specification, unless the context clearly indicates otherwise, the term "comprising" has the non-exclusive meaning of the word, in the sense of "including at least" rather than the exclusive meaning in the sense of "consisting only of". The same applies with corresponding grammatical changes to other forms of the word such as "comprise", "comprises" and so on.

It will be apparent that obvious variations or modifications may be made without departing from the scope of the invention as defined by the following claims.

**References**

Abe, T., Yasuda, T., Midorikawa, T., Sato, Y., Kearns, C. F., Inoue, K., & Ishii, N. (2005a). Skeletal muscle size and circulating IGF-1 are increased after two weeks of twice daily "KAATSU" resistance training. International Journal of KAATSU Training Research, 1(1), 6-12.

Loenneke, J. P., Wilson, G. J., & Wilson, J. M. (2010). A mechanistic approach to blood flow occlusion. Int J Sports Med, 31(1), 1-4.

Peterson, M. D., Pistilli, E., Haff, G. G., Hoffman, E. P., & Gordon, P. M. (2011). Progression of volume load and muscular adaptation during resistance exercise. European journal of applied physiology, 111(6), 1063-1071.

Estimation of limb occlusion pressure for surgical tourniquets based on the measurement of Arterial Pulse Wave Transit Time. Marko, Alexei John. 1994. Vancouver: University of British Columbia Library.

**Claims**

1. A remote controlled blood flow restriction cuff (10) comprising:

an air bladder configured to be positioned around a limb;

a compact air pump (14) located on the cuff (10) to pressurize the air bladder;

a pressure sensor (16) to sense bladder air pressure;

an electronic controller (18) to control operation of the air pump (14); the controller (18) adjustable for a predetermined pressure or pressure range, and receptive and responsive to signals from the pressure sensor (16);

a power supply (20) located on the cuff to provide power to the air pump (14), controller (18) and pressure sensor (16);

wherein inflation and deflation of the cuff can be controlled using a remote device in communication with the controller

wherein the electronic controller (18) is configured to:

inflate the cuff (10) in small increments until pulse stops;

sense at what pressure level pulse rate is no longer detected using photoplethysmography, bioimpedance, near infrared spectroscopy and/or Doppler ultrasound,

determine a limb occlusion pressure (LOP) of a user as the point at which minimal pressure is required to stop blood flow to an extremity of the limb;

inflate the cuff (10) to a desired percentage of LOP between 50-80% based on a user selection; and

to maintain the pressure at the selected percentage of LOP, keeping pressure constant in this zone allowing for adjustments with limb/body movement.

2. The restriction cuff according to claim 1 wherein the electronic controller (18) enables the cuff (10) to be inflated to and retained at a pressure between 0 and 350 mmHg.

3. The restriction cuff according to claim 1 wherein the air pump (14) is a battery operated air pump (14).

4. The restriction cuff according to claim 1 wherein the power supply (20) is a rechargeable battery power supply.

5. The restriction cuff according to claim 1 wherein the air pump (14), power supply (20), pressure sensor (16), and controller (18) are housed in a module (15) proximal to the air bladder, which are all located on the cuff (10).

6. The restriction cuff according to claim 1 wherein, the operation of the cuff (10) is controlled remotely over a wireless protocol such as Bluetooth, associated with a Smart computer or phone application (app).

7. The restriction cuff according to claim 1 wherein the pressure sensor detects pressure in the cuff (10) and the electronic controller (18) is configured to maintain the predetermined pressure or pressure range by operating the air pump (14) responsive to signals from the pressure sensor (16) detecting pressure in the cuff.

8. The restriction cuff according to claim 1 wherein the electronic controller (18) is receptive to signals from the pressure sensor (16) accounts for variance in pressure due to limb movement.

9. The restriction cuff according to claim 1 wherein the cuff (10) comprises a releasable fastening means configured to be positioned around a limb.

10. The restriction cuff according to claim 1 wherein operation of the cuff (10) is controlled with a smart phone application (app) and wherein the app includes customized programs for muscle building and strength, a timer, pressure controls, emergency stop, and tracks relevant physiological data with ability to store exercise variable data such as repetitions, sets and pressure history.

11. The restriction cuff according to claim 1 used as a medical tourniquet which allows pressure to be electronically increased and decreased via a smart phone application, wherein pressure is set at a desired level, and the pressure sensor (16) and air pump (14) make micro adjustments to keep cuff pressure constant at the desired level.

12. The restriction cuff according to claim 1 configured to be worn on a proximal upper limb (e.g. upper arm) and a proximal lower limb (e.g. upper thigh).

13. The restriction cuff according to claim 1 wherein the cuff (10) is a 5 or 10 cm cuff (10) with a ratchet, buckle, or loop Velcro fastening system.

14. The restriction cuff according to claim 1 wherein the electronic controller (18), in response to the pressure sensor (16), controls the air pump (14) to maintain pressure by inflating and deflating the cuff (10) as necessary to account for any limb movement.

15. A method of using a remote controlled blood flow restriction cuff (10) according to any one of claims 1 to 14 to build and/or strengthen muscle, the method including the steps of:

a) applying the cuff (10) to a limb of a user (40);
b) initializing and connecting the cuff (10) via a remote device or a Bluetooth protocol Smartphone application (app);
c) determining a limb occlusion pressure (LOP) of the limb of the user by inflating the cuff (10) in small increments and sensing at what pressure level a pulse rate is no longer detected using photoplethysmography, bioimpedance, near infrared spectroscopy and/or Doppler ultrasound;
d) selecting a training program and cuff pressure as a percentage of LOP of between 50-80%;
e) the user (40) sets a timer for the selected training program;
f) the user (40) presses a start button (53) of the remote device or the app;
g) the cuff (10) inflates to a desired percentage of LOP pressure;
h) the cuff (10) through an electronic controller (18) in response to a pressure sensor (16) controlling an air pump (14) which maintains pressure at the desired percentage of LOP, by inflating and deflating the cuff (10) as necessary to account for limb movement;
i) once the timer has expired or the user stops the program, the cuff (10) deflates; and
j) the remote device or the app (52) records cuff pressures and duration in a user profile.

**Patentansprüche**

1. Ferngesteuerte Blutflussrestriktionsmanschette (10), die Folgendes umfasst:

eine Luftblase, die so gestaltet ist, dass sie um eine Gliedmaße gelegt werden kann;
eine kompakte Luftpumpe (14), die sich an der Manschette (10) befindet, um die Luftblase unter Druck zu setzen;
einen Drucksensor (16) zur Messung des Luftdrucks in der Blase;
ein elektronisches Steuergerät (18) zur Steuerung des Betriebs der Luftpumpe (14); wobei das Steuergerät (18) für einen vorbestimmten Druck oder Druckbereich einstellbar ist und Signale von dem Drucksensor (16) empfängt und darauf reagiert;
eine Stromversorgung (20), die sich an der Manschette befindet, um die Luftpumpe (14), das Steuergerät (18) und den Drucksensor (16) mit Strom zu versorgen
wobei das Aufpumpen und Entleeren der Manschette über eine mit demSteuergerät kommunizierende Fernbedienungsvorrichtung gesteuert werden kann
wobei das elektronische Steuergerät (18) so konfiguriert ist, dass sie
die Manschette (10) in kleinen Schritten aufpumpt, bis der Puls stoppt
mit Hilfe der Photoplethysmographie, der Bioimpedanz, der Nahinfrarotspektroskopie und/oder des Doppler-Ultraschalls feststellt, bei welchem Druckniveau die Pulsfrequenz nicht mehr erfasst wird,
den arteriellen Okklusionsdruck (LOP) eines Benutzers als den Punkt bestimmt, an dem ein minimaler Druck erforderlich ist, um den Blutfluß zu einer Extremität der Gliedmaße zu stoppen;
die Manschette (10) auf einen gewünschten Prozentsatz des LOP zwischen 50-80% basierend auf einer Benutzerauswahl aufpumpt; und
den Druck auf dem ausgewählten Prozentsatz des LOP hält, wobei der Druck in diesem Bereich konstant gehalten wird und Anpassungen bei Bewegungen der Gliedmaßen/des Körpers möglich sind.

2. Restriktionsmanschette nach Anspruch 1, wobei die elektronische Steuerung (18) es ermöglicht, die Manschette (10) auf einen Druck zwischen 0 und 350 mmHg aufzupumpen und bei diesem zu halten

3. Restriktionsmanschette nach Anspruch 1, wobei die Luftpumpe (14) eine batteriebetriebene Luftpumpe (14) ist.

**4.** Restriktionsmanschette nach Anspruch 1, wobei die Stromversorgung (20) eine wiederaufladbare Batteriestromversorgung ist.

**5.** Restriktionsmanschette nach Anspruch 1, wobei die Luftpumpe (14), die Stromversorgung (20), der Drucksensor (16) und das Steuergerät (18) in einem Modul (15) proximal zur Luftblase untergebracht sind, die sich alle an der Manschette (10) befinden.

**6.** Restriktionsmanschette nach Anspruch 1, wobei der Betrieb der Manschette (10) über ein drahtloses Protokoll wie Bluetooth, das mit einem Smart Computer oder einer Telefonanwendung (App) verbunden ist, ferngesteuert wird.

**7.** Restriktionsmanschette nach Anspruch 1, wobei der Drucksensor den Druck in der Manschette (10) erfasst und das elektronische Steuergerät (18) so konfiguriert ist, dass es den vorbestimmten Druck oder Druckbereich aufrechterhält, indem sie die Luftpumpe (14) in Reaktion auf Signale des Drucksensors (16), der den Druck in der Manschette erfasst, betreibt.

**8.** Restriktionsmanschette nach Anspruch 1, bei der das elektronische Steuergerät (18) für Signale vom Drucksensor (16) empfänglich ist und Druckschwankungen aufgrund von Gliedmaßenbewegungen berücksichtigt.

**9.** Restriktionsmanschette nach Anspruch 1, wobei die Manschette (10) ein lösbares Befestigungsmittel umfasst, das so konfiguriert ist, dass es um eine Gliedmaße gelegt werden kann.

**10.** Restriktionsmanschette nach Anspruch 1, wobei der Betrieb der Manschette (10) mit einer Smartphone-Anwendung (App) gesteuert wird und wobei die App maßgeschneiderte Programme für Muskelaufbau und Kraft, einen Timer, Druckkontrollen, Notstopp und die Verfolgung relevanter physiologischer Daten mit der Fähigkeit, variable Übungsdaten wie Wiederholungen, Sätze und Druckverlauf zu speichern, umfasst.

**11.** Restriktionsmanschette nach Anspruch 1, die als medizinisches Tourniquet verwendet wird, das es ermöglicht, den Druck elektronisch über eine Smartphone-Anwendung zu erhöhen und zu verringern, wobei der Druck auf ein gewünschtes Niveau eingestellt wird und der Drucksensor (16) und die Luftpumpe (14) Mikroanpassungen vornehmen, um den Manschettendruck konstant auf dem gewünschten Niveau zu halten.

**12.** Restriktionsmanschette nach Anspruch 1, die so konfiguriert ist, dass sie an einer proximalen oberen Gliedmaße (z. B. Oberarm) und einer proximalen unteren Gliedmaße (z. B. Oberschenkel) getragen werden kann.

**13.** Restriktionsmanschette nach Anspruch 1, wobei die Manschette (10) eine 5 oder 10 cm lange Manschette (10) mit einem Ratschen-, Schnallen- oder Schlaufen-Klettverschluss-System ist.

**14.** Restriktionsmanschette nach Anspruch 1, bei der das elektronische Steuergerät (18) als Reaktion auf den Drucksensor (16) die Luftpumpe (14) so steuert, dass der Druck aufrechterhalten wird, indem die Manschette (10) je nach Bedarf aufgepumpt und entleert wird, um jede Gliedmaßenbewegung zu berücksichtigen.

**15.** Verfahren zur Verwendung einer ferngesteuerten Blutflussrestriktionsmanschette (10) nach einem der Ansprüche 1 bis 14 zum Aufbau und/oder zur Stärkung von Muskeln, wobei das Verfahren die folgenden Schritte umfasst:

a) Anlegen der Manschette (10) an eine Gliedmaße eines Benutzers (40);
b) Initialisierung und Verbindung der Manschette (10) über eine Fernbedienungsvorrichtung oder eine Smartphone-Anwendung (App) mit Bluetooth-Protokoll;
c) Bestimmung eines arteriellen Okklusionsdruck (LOP) der Gliedmaße des Benutzers durch Aufpumpen der Manschette (10) in kleinen Schritten und Erfassen, bei welchem Druckniveau eine Pulsfrequenz nicht mehr festgestellt wird, unter Verwendung von Photoplethysmographie, Bioimpedanz, Nahinfrarotspektroskopie und/oder Doppler-Ultraschall;
d) Auswahl eines Trainingsprogramms und eines Manschettendrucks als Prozentsatz des LOP zwischen 50-80 %;
e) der Benutzer (40) stellt einen Timer für das ausgewählte Trainingsprogramm ein;
f) der Benutzer (40) drückt eine Starttaste (53) der Fernbedienungsvorrichtung oder der App;
g) die Manschette (10) pumpt sich bis zu einem gewünschten Prozentsatz des LOP-Drucks auf;
h) die Manschette (10) durch ein elektronisches Steuergerät (18) in Reaktion auf einen Drucksensor (16) steuert eine Luftpumpe (14), die den Druck auf dem gewünschten Prozentsatz des LOP hält, indem sie die Manschette (10) nach Bedarf aufpumpt und entleert, um die Bewegung der Gliedmaßen zu berücksichtigen;

i) sobald der Zeitgeber abgelaufen ist oder der Benutzer das Programm stoppt, wird die Manschette (10) entleert; und

j) die Fernbedienungsvorrichtung oder die App (52) zeichnet Manschettendrücke und Dauer in einem Benutzerprofil auf.

**Revendications**

1. Brassard de restriction du débit sanguin télécommandé (10) comprenant :

    une vessie d'air configurée pour être positionnée autour d'un membre ;
    une pompe à air compacte (14) située sur le brassard (10) pour pressuriser la vessie d'air ;
    un capteur de pression (16) pour détecter la pression de l'air de la vessie ;
    un contrôleur électronique (18) pour contrôler le fonctionnement de la pompe à air (14) ; le contrôleur (18) est réglable pour une pression ou une plage de pression prédéterminée, et il est réceptif aux signaux du capteur de pression (16) ;
    une alimentation électrique (20) située sur le brassard pour alimenter la pompe à air (14), le contrôleur (18) et le capteur de pression (16)
    dans lequel le gonflage et le dégonflage du brassard peuvent être commandés à l'aide d'un dispositif à distance en communication avec le
    dans lequel le contrôleur électronique (18) est configuré pour
    gonfler le brassard (10) par petites étapes jusqu'à ce que le pouls s'arrête
    déterminer à quel niveau de pression la fréquence du pouls n'est plus détectée à l'aide de la photopléthysmographie, de la bioimpédance, de la spectroscopie dans le proche infrarouge et/ou de l'échographie Doppler,
    déterminer la pression d'occlusion d'un membre (LOP) d'un utilisateur comme étant le point auquel une pression minimale est nécessaire pour arrêter le flux sanguin vers une extrémité du membre ;
    gonfler le brassard (10) jusqu'à un pourcentage désiré de la LOP entre 50 et 80 % en fonction de la sélection de l'utilisateur ; et
    pour maintenir la pression au pourcentage sélectionné de la LOP, la pression restant constante dans cette zone, ce qui permet de l'ajuster en fonction des mouvements du membre ou du corps.

2. Brassard de restriction selon la revendication 1, dans lequel le contrôleur électronique (18) permet au brassard (10) d'être gonflé et maintenu à une pression comprise entre 0 et 350 mmHg

3. Brassard de restriction selon la revendication 1, dans lequel la pompe à air (14) est une pompe à air fonctionnant sur batterie (14).

4. Brassard de restriction selon la revendication 1, dans lequel l'alimentation électrique (20) est une alimentation par batterie rechargeable.

5. Brassard de restriction selon la revendication 1, dans lequel la pompe à air (14), l'alimentation électrique (20), le capteur de pression (16) et le contrôleur (18) sont logés dans un module (15) proche de la vessie d'air, qui sont tous situés sur le brassard (10).

6. Brassard de restriction selon la revendication 1 dans lequel le fonctionnement du brassard (10) est contrôlé à distance via un protocole sans fil tel que Bluetooth, associé à un ordinateur intelligent ou à une application téléphonique (app).

7. Brassard de restriction selon la revendication 1, dans lequel le capteur de pression détecte la pression dans le brassard (10) et le contrôleur électronique (18) est configuré pour maintenir la pression prédéterminée ou la plage de pression en faisant fonctionner la pompe à air (14) en réponse aux signaux du capteur de pression (16) détectant la pression dans le brassard.

8. Brassard de restriction selon la revendication 1, dans lequel le contrôleur électronique (18) est réceptif aux signaux du capteur de pression (16) et tient compte des variations de pression dues aux mouvements du membre.

9. Brassard de restriction selon la revendication 1, dans lequel le brassard (10) comprend un moyen de fixation amovible configuré pour être positionné autour d'un membre.

10. Brassard de restriction selon la revendication 1, dans lequel le fonctionnement du brassard (10) est contrôlé par une application pour téléphone intelligent (app) et dans lequel l'app comprend des programmes personnalisés pour le développement musculaire et la force, une minuterie, des contrôles de pression, un arrêt d'urgence et un suivi des données physiologiques pertinentes avec la capacité de stocker des données variables d'exercice telles que les répétitions, les séries et l'historique de la pression.

11. Brassard de restriction selon la revendication 1 utilisé comme garrot médical qui permet d'augmenter et de diminuer électroniquement la pression via une application de téléphone intelligent, dans lequel la pression est réglée à un niveau désiré, et le capteur de pression (16) et la pompe à air (14) effectuent des micro-ajustements pour maintenir la pression du brassard constante au niveau désiré.

12. Brassard de restriction selon la revendication 1 configuré pour être porté sur un membre supérieur proximal (par exemple, le bras) et un membre inférieur proximal (par exemple, la cuisse).

13. Brassard de restriction selon la revendication 1, dans lequel le brassard (10) est un brassard de 5 ou 10 cm (10) avec un système de fermeture à cliquet, à boucle ou à Velcro.

14. Brassard de restriction selon la revendication 1 dans lequel le contrôleur électronique (18), en réponse au capteur de pression (16), commande la pompe à air (14) pour maintenir la pression en gonflant et dégonflant le brassard (10) selon les besoins pour tenir compte de tout mouvement du membre.

15. Méthode d'utilisation d'un brassard de restriction du débit sanguin télécommandé (10) selon l'une des revendications 1 à 14 pour développer et/ou renforcer les muscles, la méthode comprenant les étapes suivantes :

   a) appliquer le brassard (10) sur un membre d'un utilisateur (40) ;
   b) l'initialisation et la connexion du brassard (10) par l'intermédiaire d'un dispositif à distance ou d'une application Smartphone (app) du protocole Bluetooth ;
   c) déterminer la pression d'occlusion du membre de l'utilisateur en gonflant le brassard (10) par petits paliers et en détectant à quel niveau de pression le pouls n'est plus détecté à l'aide de la photopléthysmographie, de la bioimpédance, de la spectroscopie proche infrarouge et/ou de l'échographie Doppler ;
   d) choisir un programme d'entraînement et une pression de brassard en pourcentage de la LOP comprise entre 50 et 80 % ;
   e) l'utilisateur (40) règle une minuterie pour le programme d'entraînement sélectionné ;
   f) l'utilisateur (40) appuie sur un bouton de démarrage (53) du dispositif à distance ou de l'application ;
   g) le brassard (10) se gonfle jusqu'à un pourcentage désiré de la pression LOP ;
   h) le brassard (10) par l'intermédiaire d'un contrôleur électronique (18) en réponse à un capteur de pression (16) contrôlant une pompe à air (14) qui maintient la pression au pourcentage souhaité de LOP, en gonflant et en dégonflant le brassard (10) selon les besoins pour tenir compte des mouvements du membre ;
   i) une fois que la minuterie a expiré ou que l'utilisateur a arrêté le programme, la manchette (10) se dégonfle ; et
   j) l'appareil à distance ou l'application (52) enregistre les pressions et la durée des brassards dans le profil de l'utilisateur.

FIG. 1

FIG. 2

FIG. 3

FIG. 4

FIG. 5

PRESSURE
L - 120 mmHg
R - 120 mmHg

SPO2
L - 100%
R - 100%

PULSE RATE
L - 91 bpm
R - 91 bpm

TIMER
0:59 seconds

LOP SENSOR:
ACTIVE

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017149690 A1
- US 2016193491 A1
- US 8366740 B2
- US 2016120445 A1
- US 2013211269 A1
- WO 2016148956 A1

**Non-patent literature cited in the description**

- **ABE, T.** ; **YASUDA, T.** ; **MIDORIKAWA, T.** ; **SATO, Y.** ; **KEARNS, C. F.** ; **INOUE, K.** ; **ISHII, N.** Skeletal muscle size and circulating IGF-1 are increased after two weeks of twice daily "KAATSU" resistance training. *International Journal of KAATSU Training Research*, 2005, vol. 1 (1), 6-12
- **LOENNEKE, J. P.** ; **WILSON, G. J.** ; **WILSON, J. M.** A mechanistic approach to blood flow occlusion. *Int J Sports Med*, 2010, vol. 31 (1), 1-4
- **PETERSON, M. D.** ; **PISTILLI, E.** ; **HAFF, G. G.** ; **HOFFMAN, E. P.** ; **GORDON, P. M.** Progression of volume load and muscular adaptation during resistance exercise. *European journal of applied physiology*, 2011, vol. 111 (6), 1063-1071
- **MARKO, ALEXEI JOHN**. Estimation of limb occlusion pressure for surgical tourniquets based on the measurement of Arterial Pulse Wave Transit Time. University of British Columbia Library, 1994